# EUROPÄISCHE PATENTSCHRIFT

(11) **EP 1 021 986 B1**
(45) Veröffentlichungstag und Bekanntmachung des Hinweises auf die Patenterteilung: **11.05.2005**
(21) Anmeldenummer: 99121446.1
(22) Anmeldetag: 28.10.1999
(51) Int. Cl.: A61B 5/0408, A61N 1/04

(54) **Biomedizinische Einmalelektrode und Verfahren zu deren Herstellung**
Disposable biomedical electrode and method of manufacture
Electrode biomédicale jetable et procédé de fabrication

(30) Priorität: 29.10.1998 DE 29819246 U; 27.04.1999 DE 19918963
(43) Veröffentlichungstag der Anmeldung: 26.07.2000
(73) Patentinhaber: Tyco Healthcare Deutschland Manufacturing GmbH, 93333 Neustadt/Donau (DE)
(72) Erfinder: Anz, Johannes, 38820 Halberstadt (DE); Malkowsky, Andrea, 38828 Wegeleben (DE); Kappe, Heimo, 38820 Halberstadt (DE)
(74) Vertreter: Tragsdorf, Bodo, Dipl.-Ing.

(56) Entgegenhaltungen:
- DE-A- 3 215 960
- DE-A- 4 336 300
- US-A- 4 362 165
- US-A- 4 474 570
- US-A- 4 819 328

## Beschreibung

Die Erfindung betrifft eine biomedizinische Einmalelektrode, die zur Übertragung elektrischer Signale von der Haut zu einem elektromedizinischen Gerät (Passivelektroden) oder zur Übertragung externer elektrischer Signale zur Haut (Aktivelektroden) eingesetzt wird, und ein Verfahren zur Herstellung der Einmalelektrode.

Bekannt sind Elektroden mit einem Gelkörper aus einem klebenden und elektrisch leitenden Material, vorzugsweise einem Polymer. Der Gelkörper steht nach der Applikation der Elektrode mit der Haut des Patienten in direktem Berührungskontakt. Der Gelkörper kann aus einer in situ polymerisierbaren oder aushärtbaren Reaktionsmischung bestehen, die im Ausgangszustand fließfähig ist und erst während der Herstellung, z.B. durch UV-Licht-Einwirkung, polymerisiert wird. Erst nach erfolgter Polymerisation wird der sogenannte Leitgelkörper gebildet, der elastisch verformbar ist. Zum Auftragen der Reaktionsmischung für den Gelkörper ist es erforderlich, in dem dafür vorgesehenen Bereich des Trägermaterials eine Kavität zu bilden. Hierzu ist bekannt, daß bei einer Ausführung des Trägermaterials aus thermoplastischem Material die Kavität durch eine trogförmige Verformung oder durch einen separaten trogförmigen Kunststoffeinsatz, der in eine dafür vorgesehene Aussparung des Trägermaterials eingesetzt und mit dem Trägermaterial durch Kleben verbunden wird, gebildet wird (DE 43 36 302 A1). Besteht das Trägermaterial aus einer geschäumten Kunststoff- oder Vliesstoffbahn, so kann die Kavität zur Aufnahme der polymerisierbaren Reaktionsmischung für den Leitgelkörper durch Ausstanzen einer Aussparung in der Bahn, die von unten mit einer aufgeklebten Folie abgedeckt wird, und durch Tiefziehen der Folie, gebildet werden (DE 43 36 300 C2).
Aus der US-A-4 819 328 ist eine Elektrode bekannt, die aus einem Trägermaterial besteht, an dessen zur Haut zeigender Seite ein dehn- bzw. streckbares, elektrisch leitendes Gestrick mittels eines Klebers befestigt ist und die Zwischenräume des leitenden Gestricks mit einem flexiblen, elektrisch leitenden Kleber ausgefüllt sind und der den Leitgelkörper bildende Kleber das Gestrick an der zur Haut zeigenden Seite in einer definierten Schichtdicke überragt. Der Leitgelkörper ist mit einer unmittelbar vor der Applikation der Elektrode abnehmbaren Abdeckung versehen. Diese Elektrode soll aufgrund des dehnbaren leitenden Gestricks und des flexiblen leitenden Klebers in der Lage sein, sich mit der Haut des Patienten zu bewegen, um eine richtige dauerhafte Anordnung der Elektrode in Bezug auf das zu stimulierende Nerven- oder Muskelgewebe sicherzustellen und um eine lange haltende, kontinuierliche elektrische Verbindung zu gewährleisten, ohne die Haut zu irritieren oder den zu behandelnden Patienten zu beeinträchtigen. Zur Herstellung dieser Elektrode wird erst ein Schichtaufbau, bestehend aus dem Trägermaterial, dem leitenden Gestrick, dem leitenden Kleber und der entfernbaren Abdeckung, gebildet, aus dem die einzelnen Elektrodenformkörper ausgestanzt und anschließend durch Anbringung des elektrischen Anschlusselementes konfektioniert werden.
Weiterhin ist eine Elektrode bekannt (US-A-4 362 165), bestehend aus einem Schaumring, einem im Innenraum des Schaumringes angeordnetem Gelkörper sowie einer in dem Gelkörper angeordneten leitfähigen Einlage mit einem nach außen über den Rand des Schaumringes ragenden Anschlusselement und einer Kunststoffabdeckung an der von der Haut abgewandten Oberseite. Der Gelkörper ist an der zur Haut zeigenden Seite nach außen gewölbt. Die Herstellung der Elektrode erfolgt in einem Kunststoffbehälter aus PP oder PE, der zugleich auch als Lagerbehälter dient.
Aus der EP 0 627 193 ist eine Elektrode bekannt, die aus einem Trägermaterial besteht, dessen hautzugewandte Seite mit einem hautverträglichen Kunststoff beschichtet ist, auf der eine in ihrer Außenkontur kleinere Verstärkungsfolie aufgeklebt ist. Durch die zentrale Öffnung des Trägermaterials und der Verstärkungsfolie ist das Innenteil eines zweiteiligen Druckknopfanschlußelementes gesteckt und das Außenteil in an sich bekannter Weise montiert. Der Gelkörper ist auf einer Trägerschicht aufgetragen und als vorgefertigte Baueinheit auf der Verstärkungsfolie aufgeklebt und steht in direktem Kontakt mit dem Basisteil des Innenteils des Druckknopfanschlußelementes. Die auf die Haut des Patienten zeigende Gelkörperfläche und der Klebrand des Trägermaterials sind mit einer abziehbaren Abdeckung abgedeckt. Bei einer Ausführung mit einem einteiligen Druckknopfanschlußelement ist es erforderlich, noch eine Verstärkungsschicht einer relativ steifen Kunststoffscheibe, z.B. aus Polyester, anzuordnen, um das einteilige Druckknopfelement zu halten. Diese in ihrem Aufbau sehr einfache Elektrode hat den Nachteil, daß der Gelkörper mit der Trägerschicht gesondert herzustellen ist. Um eine ausreichende Haftung des Gelkörpers an der Verstärkungsfolie mit dem Druckknopfinnenteil zu gewährleisten, muß der Gelkörper eine relativ große Fläche aufweisen. Bei Patienten mit starker Schweißbildung und bei längeren Tragezeiten der Elektrode besteht die Gefahr, daß der Gelkörper nicht mehr in ausreichendem Maße haftet und Signalübertragungsfehler auftreten. Ferner sind aus der Praxis Elektroden bekannt, bei denen der Gelkörper in einem sogenannten Gelteller aus hochgefülltem Polyethylen mit Kreide als Füllstoff gehalten wird. Bei diesen Elektroden trat nach einer längeren Lagerung eine Blasenbildung auf, die vermutlich auf Reaktionen zwischen dem Füllstoff des Geltellers und der üblicherweise verwendeten AgCl-Schicht des Sensors zurückzuführen ist. Dadurch wird die Signalübertragungsqualität so beeinträchtigt, daß die Elektroden unbrauchbar sind. Die bekannten Elektroden sind in ihrer Herstellung noch zu aufwendig, insbesondere die Ausbildung einer Kavität zur Aufnahme der Reaktionsmischung für den Gelkörper erfordert in technologischer Hinsicht zusätzliche Arbeitsschritte, verbunden mit einem erhöhten maschinentechnischen Aufwand.

Der Erfindung lag die Aufgabe zugrunde, eine gattungsgemäße Elektrode in ihrem Aufbau so zu verbessern, daß eine kostengünstige Herstellung ermöglicht wird und die störende Ablösung des Leitgelkörpers aus dem Gesamtaufbau der Elektrode während der Applikation oder spätestens bei Entfernung der Elektrode von der Haut sowie unerwünschte Reaktionen des Leitgelkörpers während einer längeren Lagerungsdauer der Elektroden vermieden werden. Ferner soll ein Verfahren zur Herstellung der Elektroden geschaffen werden.

Erfindungsgemäß wird die Aufgabe durch die in den Ansprüchen 1 oder 2 angegebenen Merkmale gelöst. Geeignete Ausgestaltungsvarianten der Elektroden sind in den Ansprüchen 3 bis 7 angegeben. Die Verfahrensweise zur Herstellung der Elektroden ist Gegenstand der Ansprüche 8 oder 9. Geeignete Ausgestaltungen in Bezug auf die Herstellung der Elektroden sind in den Ansprüchen 10 bis 14 angegeben.
Wesentliches Merkmal der neuen Elektrode ist die Anordnung einer vliesartigen planen Unterlage, auf deren hautzugewandter Seite der Gelkörper aufgetragen ist, wobei die Gelkörperausbreitung durch die äußere Kontur der vliesartigen Unterlage begrenzt ist. Die vliesartige Unterlage bildet somit das Trägerelement für den Gelkörper. Die ansonsten üblichen Arbeitsschritte zur Bildung einer Kavität, z.B. durch Tiefziehen oder Ausstanzen einer Öffnung und deren Abdeckung von einer Seite mittels einer zusätzlichen Folie, können somit entfallen. Dadurch kann der technologische Herstellungsprozeß für die Elektroden wesentlich vereinfacht und die Gestehungskosten für die Elektroden gesenkt werden. Sie besteht vorzugsweise aus organischen Kunst- oder Naturfasern oder aus Mischungen dieser Fasern, wobei die Kunst- oder Naturfasern aus Polypropylen, Polyester, Kunstseide, Baumwolle oder Papier hergestellt werden.
Das Trägermaterial kann aus einem flexiblen Flächengebilde, wie einer geschlossenzelligen Polymerschaumbahn, einem textilen Flächengebilde aus Gewebe oder Vlies oder einer kompakten Kunststoffolie bestehen, dessen hautzugewandte Seite mit einem biokompatiblen Haftklebstoff beschichtet ist.
Erforderlichenfalls kann das Trägermaterial elektrisch leitfähig ausgebildet sein und auf der zur Haut abgewandten Seite mit einer Isolierschicht versehen sein. Ist das Trägermaterial aus einer Kunststoffolie gebildet, so kann deren hautzugewandte Seite mit einem kurzfaserigen Material beflockt werden.
Das Trägermaterial sollte zumindest an der der Haut zugewandten Seite eine plane Fläche aufweisen, auf die die vliesartige Unterlage aufgeklebt wird. Das Trägermaterial ist in der Regel an der zur Haut zeigenden Seite ganzflächig mit einem hautverträglichen Klebstoff beschichtet. Die vliesartige Unterlage ist in ihrer äußeren Kontur bereits auf die Grundfläche des zu bildenden Gelkörpers abgestimmt. Nach dem Aufkleben der vliesartigen Unterlage auf das Trägermaterial wird auf diese die Reaktionsmischung für den Leitgelkörper aufgetragen. Infolge der Struktur der vliesartigen Unterlage und im besonderen, im Falle einer zusätzlichen Benetzung der vliesartigen Unterlage mit einer geeigneten Flüssigkeit, breitet sich die Reaktionsmischung für den Leitgelkörper nur über die gesamte Oberfläche der vliesartigen Unterlage aus, und gelangt nicht auf den benachbarten Bereich des Trägermaterials. Bei besonders niedrigviskosen Mischungen zur Bildung des Gelkörpers als Hydrogel kann es zweckmäßig sein, die vliesartige Unterlage entweder mit einer parallel zur Außenkontur angeordneten oder mit der Außenkontur verlaufenden, umlaufenden Erhebung auszurüsten, was z.B. durch Einformung einer Sicke auf der gelkörperabgewandten Seite der Unterlage erfolgen kann.
Die Reaktionsmischung wird dann in an sich bekannter Weise, z.B. durch UV-Strahlung, zur Polymerisation und Vernetzung gebracht.
Dieser Verfahrensschritt ist integrierter Bestandteil einer taktweise oder kontinuierlich arbeitenden vollautomatischen Fertigungslinie. Der Gelkörper kann ausgehend von den flüssigen Reaktionsmischungen durch Vernetzung mittels Strahlung oder Wärme gebildet werden, wie z.B. bei Gelkörpern auf der Basis von Acrylaten, oder bei Gelkörpern auf der Basis von Polyalkoholen durch Verfestigung infolge von Wasserstoffbrückenbildung. Gele auf der Basis gelierender Substanzen können z.B. aus der Schmelze erhalten werden. Die vliesartige Unterlage sollte ein Flächengewicht von 4 bis 250 g/m², vorzugsweise von 14 bis 48 g/m² aufweisen und thermisch oder mittels Bindemitteln verfestigt sein. Außerdem sollte diese in Abhängigkeit von der Zusammensetzung der Reaktionsmischung flüssigkeits- und UV-strahlungsdurchlässig sein. Infolge der vliesartigen Struktur der Unterlage wird zwischen dieser und der aufgetragenen Reaktionsmischung eine besonders gute Haftung erzielt. Von Vorteil ist es, wenn die Seite der vliesartigen Unterlage, auf die die Reaktionsmischung aufgetragen wird, vor dem Auftragen der Reaktionsmischung geglättet wird. Dadurch wird verhindert, daß möglicherweise nach Abschluß der Gelbildung noch Vliesfasern aus dem Gelkörper herausragen.
Es besteht sogar die Möglichkeit, als Unterlage vliesartig ausgebildetes Papier einzusetzen, wodurch eine weitere Kostenreduzierung erreicht werden kann. Das elektrische Anschlußelement kann verschiedenartig ausgebildet werden. Bei einem zweiteiligen Druckknopf als elektrisches Anschlußelement kann es erforderlich sein, auf die der Haut zugewandten Seite des Trägermaterials, um den Bereich des Druckknopfes, eine steife Zwischenlage anzuordnen, die dazu dient, dem Trägermaterial, z.B. einer geschlossenzelligen Polymerschaumfolie, eine höhere Festigkeit gegen ein Ausreißen des Druckknopfes zu verleihen. Eine Versteifung der Elektrode im Gelbereich reduziert Signalstörungen, die infolge von außen aufgebrachter mechanischer Kräfte entstehen können. Die steife Zwischenlage sollte mindestens in ihrer Fläche so groß sein, wie der Gelkörper bzw. die vliesartige Unterlage zur Aufnahme des Gelkörpers und aus einem gegen Gelbestandteile beständigem Material bestehen. Bei einer notwendigen Anordnung einer steifen Zwischenlage wird die vliesartige Unterlage auf der zur Haut zeigenden Seite der Zwischenlage aufgeklebt. Die vliesartige Unterlage und die Zwischenlage können auch aus einem Teil bestehen. Falls keine Zwischenlage erforderlich ist, so wird die vliesartige Unterlage direkt auf die zur Haut zeigende Seite des Trägermaterials aufgeklebt. Die Elektrode kann in an sich bekannter Weise mit einer klebstofffreien Grifflasche ausgerüstet werden, um die auf der hautzugewandten Seite befindliche Abdeckung vor der Applikation der Elektrode zu entfernen.
Die Anordnung der vliesartigen Unterlage zur Aufnahme der Mischung für die Gelkörperbildung wirkt sich äußerst vorteilhaft auf die Herstellungstechnologie aus. Die hergestellten Elektroden zeichnen sich durch sehr gute Trageigenschaften, auch bei längerer Applikationsdauer, aus und erfüllen die Anforderungen an eine hohe Signalübertragungsqualität.
Als elektrisch leitendes Kontaktelement kann ein Litzenanschluß eingesetzt werden, wobei ein freies Ende der Litze zum Beispiel mit einem Lötkontakt verbunden wird, der in eine zentrale Öffnung des Trägermaterials eingesetzt wird und auf der zur Haut zeigenden Seite des Trägermaterials ein flächenförmiger Sensor aufgeklebt wird, der sich zwischen der vliesartigen Unterlage und dem Trägermaterial befindet, und in Berührungskontakt mit dem Leitgelkörper und dem Lötkontakt steht.
Das elektrisch leitende Anschlußelement kann auch aus einer Ag/AgCl-beschichteten Kunststoffscheibe mit einer angeformten Nase, die durch das Trägermaterial gesteckt wird bestehen, wobei die Nase mit einer Öffnung zur Aufnahme und Befestigung einer Litze versehen ist.
Als Trägermaterial können eine transparente Kunststoffolie und als Anschlußelement eine röntgentransluzentes Bauteil eingesetzt werden.
Die vliesartige Unterlage kann so ausgebildet werden, daß durch diese die gesamte Elektrodenfläche klebrandfrei bedeckt wird.

Die Erfindung soll nachstehend an mehreren Beispielen unterschiedlicher Elektrodenbauarten erläutert werden. In der zugehörigen Zeichnung zeigen
- Fig. 1: eine Schaumelektrode mit einem Druckknopfanschluß als Draufsicht,
- Fig. 2: eine Explosionsdarstellung der Elektrodenbauteile der Elektrode gemäß Fig. 1,
- Fig. 3: eine Neonatenelektrode mit Litzenanschluß als Draufsicht,
- Fig. 4: eine Explosionsdarstellung der Elektrodenbauteile der Elektrode gemäß Fig. 3,
- Fig. 5: eine Neutralelektrode als Draufsicht und
- Fig. 6: eine Explosionsdarstellung der Elektrodenbauteile der Elektrode gemäß Fig. 5.

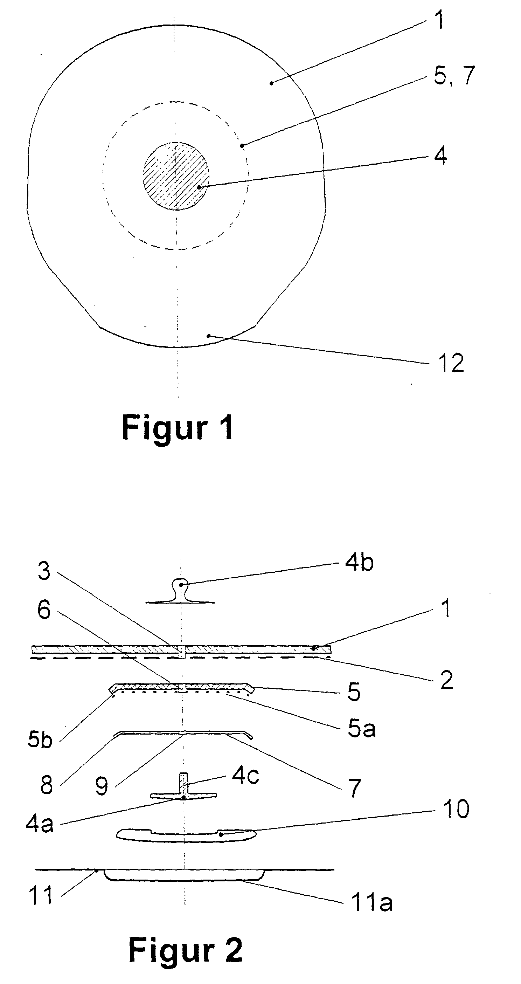

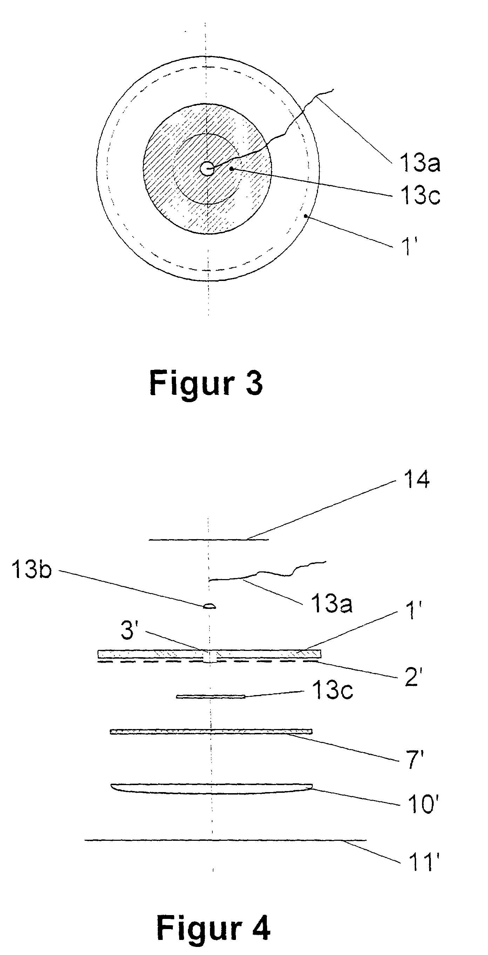

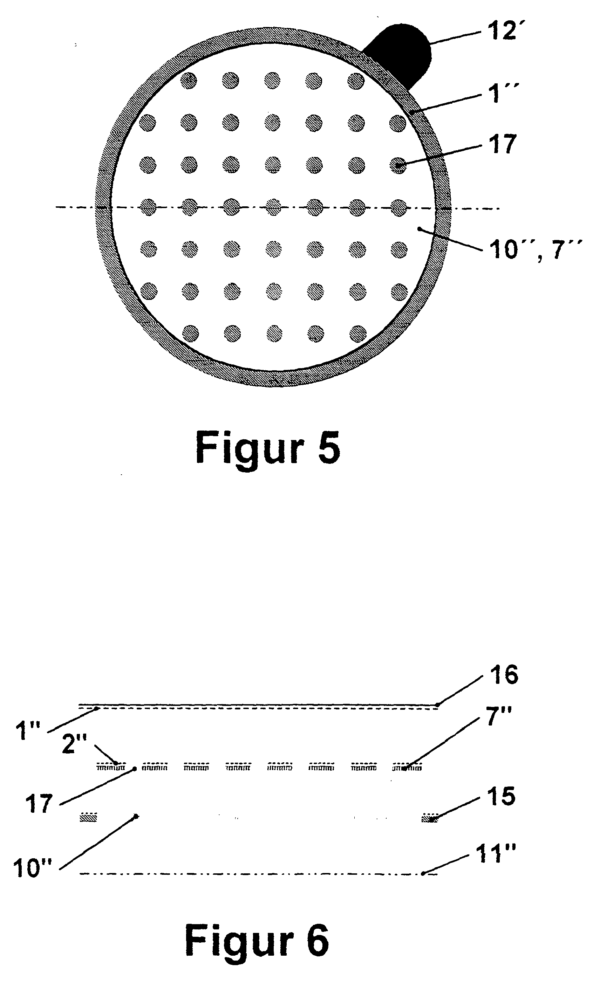

Die in den Figuren 1 und 2 dargestellte Schaumelektrode besteht aus einem Trägermaterial 1 aus einer handelsüblichen geschlossenzelligen Polymerschaumbahn. Die Polymerschaumbahn ist an der zur Haut zugewandten Seite ganzflächig mit einem biokompatiblen, hautverträglichen Klebstoff 2 beschichtet und weist eine zentrale Öffnung 3 für die Montage eines zweiteiligen Druckknopfanschlußelementes 4 auf, das aus einem Innenteil 4a und einem Außenteil 4b besteht. Das Innenteil 4a, der sogenannte Nietsensor, besteht aus fasergefülltem ABS, überzogen mit einer Ag/AgCl-Schicht. Das auf den Zapfen 4c des Innenteils 4a aufsteckbare Außenteil 4b besteht aus Edelstahl. Eine steife Zwischenlage 5, die aus Kunststoff oder auch aus Papier bestehen kann, ist mit einer zentralen Öffnung 6 versehen, die den gleichen Durchmesser wie die Öffnung 3 aufweist. Die Zwischenlage 5 ist in ihrem Außendurchmesser mindestens so groß wie der spätere Gelkörper 10. Auf der zur Haut zeigenden Seite der Zwischenlage 5, die mit Klebstoff 5a beschichtet ist, wird eine vliesartige Unterlage 7 aufgeklebt, die in ihrer Außenkontur deckungsgleich mit der steifen Zwischenlage 5 ist. Die vliesartige Unterlage 7 besteht aus kalandriertem Cellulosevlies und besitzt ein Flächengewicht von 38 g/m². Dieses Vlies wird in einem getrennten Verfahrensschritt mittels Klebstoff 5a auf eine Zwischenlage 5 aus einer Kunststoffbahn flächig aufgebracht, woraus dann im Verfahren der Elektrodenmontage eine Gelunterlage ausgeschnitten und auf die Haftklebstoffseite 2 des Trägermaterials 1 aufgesetzt wird. Gleichzeitig wird eine umlaufende Erhebung 8 in diese Unterlage geformt. Die jeweiligen Öffnungen 3, 6 und 9 decken sich durch das anschließende Einbringen dieses Ausschnittes. Die Zwischenlage 5 kann in analoger Weise wie die Unterlage 7 mit einer umlaufenden Erhebung 5b versehen sein. Anschließend wird der Zapfen 4c des Innenteils 4a des Druckknopfes durch die Öffnungen 3, 6 und 9 gesteckt und auf den aus dem Trägermaterial 1 herausragenden Zapfen 4c des Außenteils 4b aufgesteckt und beide Teile innig miteinander verbunden. Die vliesartige Unterlage wird mit einer Flüssigkeit benetzt und anschließend die flüssige Reaktionsmischung für den Gelkörper 10 aufgetragen. Infolge der Vliesstruktur und deren Benetzung breitet sich die Reaktionsmischung nur bis an den äußeren Rand der vliesartigen Unterlage 7 aus. Die Reaktionsmischung gelangt dabei in innigen Kontakt mit dem Nietsensor und durchdringt die den Nietsensor angrenzenden Bereiche der vliesartigen Unterlage. Dadurch wird eine besonders gute Haftverbindung zwischen dem Vlies und dem Gelkörper 10, nach dessen Aushärtung mittels UV-Initiierung, erzielt. Die Zusammensetzungen geeigneter Reaktionsmischung sind z.B. in der DE 43 36 300 C2 angegeben.
Nach Abschluß der Polymerisationsreaktion wird auf die zur Haut zeigende Seite der Elektrode eine Abdeckung 11 mit Antihaftausrüstung aufgeklebt. Diese besitzt eine trogförmige Ausnehmung, da der Gelkörper 10 nicht plan mit der Klebfläche 2 des Trägermaterials 1 abschließt. Vor der Applikation der Elektrode wird die Abdeckung abgenommen, wobei hierfür eine Abgrifflasche 12 vorgesehen ist.
In Abhängigkeit von der Materialbeschaffenheit des Trägermaterials kann auch auf die Anordnung der Zwischenlage 5 verzichtet werden. Durch die Zwischenlage soll lediglich ein Ausreißen des Druckknopfanschlusses verhindert werden. Bei einer Ausführung ohne die Zwischenlage 5 wird die vliesartige Unterlage direkt auf das Trägermaterial 1 aufgeklebt. Die vliesartige Unterlage kann auch aus einer relativ steifen Kunststofflage bestehen, deren hautzugewandte Seite durch Beflocken mit kurzen Polyesterfasern mit einer Länge von ca. 1 mm beschichtet ist. Besteht die vliesartige Unterlage aus Papier, so sollte deren Flächengewicht mindestens 120 g/m² betragen. Die hautzugewandte Seite der Papierunterlage besitzt eine faserige Oberflächenstruktur. Die Zusammensetzung des Papiers muß so beschaffen sein, daß eine unzulässige Reduzierung der AgCl-Schicht an der Oberfläche des Ableitsensors vermieden wird. Ein entsprechend geeignetes Papier ist z.B. frei von reduzierenden Gipsbestandteilen und hat ein Flächengewicht von mindestens 120 g/m². Anstelle eines Druckknopfanschlusses kann auch ein Ag/AgCl-beschichtetes Kunststoffteil eingesetzt werden, das analog wie das Innenteil eines Druckknopfanschlusses ausgebildet ist. Die aus dem Trägermaterial herausragende Nase besitzt eine Öffnung, z.B. einen Schlitz, so daß nach der Montage des Sensors eine Litze angeschlagen werden kann, z.B. durch Ultraschall- oder thermisches Schweißen. Der beim konventionellen Montageverfahren von Gelkörpern aus vorgefertigter Bahnenware auftretende Nachteil, daß die Geloberfläche sich über dem Sensor aufwölbt und das Gel somit schlechter auf der Haut haftet, tritt hier nicht auf, da das Gel bei der anschließenden in situ-Verarbeitung derartige Unebenheiten ausgleicht.
Die in den Figuren 1 und 2 gezeigte Elektrode kann auch in röntgentransluzenter sowie optisch transparenter Ausführung hergestellt werden. Als Trägermaterial wird eine transparente Kunststoffolie, z.B. aus Polypropylen, mit einer Dicke von ca. 80 µm eingesetzt, die an der hautzugewandten Seite mit Haftklebstoff beschichtet wird. Als elektrisches Anschlußelement wird an Stelle des Druckknopfanschlusses ein Kunststoffteil, wie vorstehend erläutert, in röntgentransluzenter Ausführung verwendet. Nach dessen Montage wird eine vliesartige Unterlage mit einem Flächengewicht von ca. 38 g/m² mittig über dem Sensor bzw. Kunststoffteil fixiert. Die weiteren Verfahrensschritte entsprechen denen, die bereits bei der Beschreibung zu den Figuren 1 und 2 angegeben sind.
Eine solche Elektrode ist durch ihren Aufbau sowohl röntgentransluzent als auch optisch transparent, ausgenommen der Sensorbereich, und hat den Vorteil, daß diese bei notwendigen Röntgenuntersuchungen des Patienten nicht abgenommen werden muß. Außerdem ermöglicht diese die visuelle Wahrnehmung möglicher
Hautreaktionen während der Applikation der Elektrode. Die Herstellung dieser Elektrode ist im Vergleich zu anderen bekannten Elektroden sehr einfach und verursacht nur geringe Kosten.
Die in den Figuren 3 und 4 gezeigte Neonatenelektrode unterscheidet sich in ihrem Aufbau von der in den Figuren 1 und 2 gezeigten Elektrode im wesentlichen durch ein anderes Anschlußelement, und anwendungsbedingt wird auf einen Klebrand verzichtet. Das Trägermaterial 1' besteht aus einem flexiblen Flächenmaterial, z.B. einem Mischvlies aus Viskose- und Kunstseidefasern mit einem Flächengewicht von 35 g/m², welches eine kleine Öffnung 3' für den Litzenanschluß 13 aufweist. An der zur Haut zeigenden Seite ist auf dem Trägermaterial 1' ganzflächig eine Klebstoffschicht 2' aufgetragen. Der Litzenanschluß 13 besteht aus einer Litze 13a, einem an dem einen Litzenende befestigten Lötkontakt 13b zur Signalableitung und einem Sensor 13c aus Silberblech, 0,15 mm dick, das gelseitig chloriert ist. Der Lötkontakt 13b befindet sich in der Öffnung 3' des Trägermaterials 1' und steht mit dem aufgeklebten Sensor 13c in Berührungskontakt. Die vom Lötkontakt 13b nach außen wegführende Litze 13a ist im Bereich um den Lötkontakt mit einer elektrisch isolierenden Abdeckung 14 versehen, die auf der hautabgewandten Seite des Trägermaterials 1' aufgeklebt ist. Auf der zur Haut zeigenden Seite des Trägermaterials 1' ist eine vliesartige Unterlage 7' aufgeklebt, die in ihrer Außenkontur nahezu deckungsgleich mit dem Trägermaterial 1' ist und den Sensor 13 c vollständig überdeckt.
Die vliesartige Unterlage 7' besteht aus Polypropylenvlies und besitzt ein Flächengewicht von 27 g/m². Auf die mit einer Flüssigkeit benetzte vliesartige Unterlage 7' wird in analoger Weise, wie bei der Erläuterung zu Fig. 1 und 2 beschrieben, die Reaktionsmischung zur Bildung des Gelkörpers 10' aufgetragen. Die Reaktionsmischung verteilt sich über das Vlies 7', durchdringt die offenen Stellen der Vliesstruktur und gelangt dadurch in Berührungskontakt mit dem Sensor 13c. Nach Beendigung der Polymerisation wird die zur Haut zeigende Seite der Elektrode mit einer Abdeckung 11' versehen, die vor der Applikation entfernt wird.
Eine weitere Ausführungsvariante der Elektrode für den Anwendungsbereich der Stromtherapie, z.B. zur Muskelstimulation oder als sogenannte Neutralelektrode in der HF-Chirugie, ist in den Figuren 5 und 6 gezeigt. Dabei handelt es sich um großflächige Elektroden. Über Stromdichtelimitierung und -verteilung werden örtliche Hautverbrennungen vermieden. Im Unterschied zu den vorgenannten Elektrodenausführungen ist das Trägermaterial 1" elektrisch leitfähig ausgebildet. Als Trägermaterial 1" sind hierfür flexible Aluminiumfolien oder auch leitfähige, flexible Polymerfolien geeignet. Das leitfähige Trägermaterial 1" ist auf der hautabgewandten Seite durch eine aufgebrachte Isolierschicht 16 geschützt, wobei in bestimmten Anwendungsfällen auch auf eine Isolierschicht verzichtet werden kann. Auf der hautzugewandten Seite des Trägermaterials 1" wird eine vliesartige Unterlage 7" mittels eines Haftklebstoffes befestigt, wobei die vliesartige Unterlage 7" bereits mit einer selbsthaftenden Klebstoffschicht 2" ausgerüstet ist, um einen kraftschlüssigen Verbund zum elektrisch leitfähigen Trägermaterial zu erreichen. Die vliesartige Unterlage 7" ist mit über die Fläche verteilten Durchbrüchen 17 versehen. Nach dem Auftragen der Reaktionsmischung für den Leitgelkörper 10" und dessen Aushärtung befindet sich in den Durchbrüchen 17 Leitgelkörpermaterial, durch das die elektrische Verbindung zwischen dem leitfähigen Trägermaterial 1" und dem Leitgelkörper 10" hergestellt wird. Die zur Haut zeigende Seite der Elektrode wird in an sich bekannter Weise mittels einer abnehmbaren Abdeckung 11", z. B. einer silikonisierten Kunststoffolie, abgedeckt. Am Rand des leitfähigen Trägermaterials 1" ist ein umlaufender Schaumstoffstreifen 15 aufgeklebt, durch den der direkte elektrische Kontakt zur Haut über die Schnittkante des leitfähigen Trägermaterials 1" verhindert wird. Die Hauthaftung dieser Elektrode erfolgt analog wie bei der Ausführungsvariante gemäß den Figuren 3 und 4 ausschließlich über den selbsthaftenden Leitgelkörper 10". Der elektrische Anschluß der Elektrode erfolgt über eine seitliche Lasche 12', die auf der hautzugewandten Seite elektrisch isoliert ist. Die Lasche 12' besteht aus dem gleichen Werkstoff wie das Trägermaterial 1". An der Lasche 12' wird mittels Hohlniet eine nicht näher dargestellte Litze angeschlagen. Auch diese Elektrode zeichnet sich durch eine kostengünstige Herstellung aus.

## Patentansprüche

1. Biomedizinische Einmalelektrode, bestehend aus mindestens einem, in einer Fertigungslinie in fließfähiger Konsistenz aufgebrachten und anschließend verfestigten Leitgelkörper (10, 10', 10"), der nach der Applikation der Elektrode auf der Haut des Patienten aufliegt, einem den Leitgelkörper (18, 10', 10") abdeckenden Trägermaterial (1, 1'), dessen zur Haut zeigende Seite plan ausgebildet ist, einer entfernbaren Abdeckung (11, 11', 11 ") an der der Haut zugewandten Seite der Elektrode und einem mit dem Leitgelkörper (10, 10', 10") in Verbindung stehenden elektrischen Anschlusselement (4, 13), **gekennzeichnet durch** eine mit dem Trägermaterial (1, 1') verbundene vliesartige plane Unterlage (7, 7', 7") auf deren hautzugewandter Seite zur Bildung des Gelkörpers (10, 10', 10") eine Reaktionsmischung in fließfähiger Konsistenz aufgebracht ist, wobei die Viskosität der Reaktionsmischung derart hoch ist, dass deren Ausbreitung **durch** die äußere Kontur der vliesartigen Unterlage (7, 7',7") begrenzt ist.

2. Biomedizinische Einmalelektrode, bestehend aus mindestens einem, in einer Fertigungslinie in fließfähiger Konsistenz aufgebrachten und anschließend verfestigten Leitgelkörper (10, 10', 10"), der nach der Applikation der Elektrode auf der Haut des Patienten aufliegt, einem den Leitgelkörper (10, 10', 10") abdeckenden Trägermaterial (1, 1'), dessen zur Haut zeigende Seite plan ausgebildet ist, einer entfernbaren Abdeckung (11, 11', 11") an der der Haut zugewandten Seite der Elektrode und einem mit dem Leitgelkörper (10, 10', 10") in Verbindung stehenden elektrischen Anschlusselement (4, 13), **gekennzeichnet durch** eine mit dem Trägermaterial (1,1') verbundene vliesartige plane Unterlage (7, 7', 7") auf deren hautzugewandter Seite zur Bildung des Gelkörpers (10, 10', 10") eine Reaktionsmischung in fließfähiger Konsistenz aufgebracht ist, deren Ausbreitung **durch** die äußere Kontur der vliesartigen Unterlage (7, 7',7") begrenzt ist, wobei die vliesartige Unterlage (7, 7', 7") eine parallel zur Außenkontur angeordnete oder mit der Außenkontur verlaufende, umlaufende Erhebung (8) besitzt.

3. Einmalelektrode nach einem der Ansprüche 1 oder 2, **dadurch gekennzeichnet, daß** die vliesartige Unterlage (7, 7', 7") aus organischen Kunst- oder Naturfasern oder aus Mischungen dieser Fasern oder aus cellulosehaltigem Material, wie Papier oder Pappe, oder einer einseitig beflockten Kunststofflage besteht, wobei deren vorstehende freie Fasern im Gelkörper (10, 10', 10") fest verankert sind, und die vliesartige Unterlage (7, 7', 7") ein Flächengewicht von 4 bis 250 g/m² aufweist und thermisch oder mit Hilfe von Bindemitteln verfestigt ist sowie gelseitig mindestens teilweise flüssigkeit- und UV-strahlendurchlässig ist.

4. Einmalelektrode nach einem der Ansprüche 1 bis 3, **dadurch gekennzeichnet, daß** das Trägermaterial (1, 1', 1") aus einer geschlossenzelligen Polymerschaumfolie, einem textilen Flächengebilde aus Gewebe oder Vlies oder einer kompakten Kunststoffolie besteht, dessen hautzugewandte Seite mit einem biokompatiblen Haftklebstoff (2, 2', 2") beschichtet ist.

5. Einmalelektrode nach einem der Ansprüche 1 bis 4, **dadurch gekennzeichnet, daß** die vliesartige Unterlage (7, 7', 7") mit einer Zwischenlage (5) kraftschlüssig thermisch und/oder mittels eines Bindemittels fest verbunden ist, und der Verbund auf der hautzugewandten Seite des Trägermaterials (1) befestigt ist, und die Zwischenlage (5) aus Papier, Pappe oder Kunststoff besteht und mit einer umlaufenden Erhebung (5b) versehen ist, die paßgenau auf die Erhebung (8) der vliesartigen Unterlage (7) abgestimmt ist.

6. Einmalelektrode nach einem der Ansprüche 1 bis 5, **dadurch gekennzeichnet, daß** das Anschlußelement
a) als zweiteiliger Druckknopfanschluß (4), bestehend aus einem Außenteil (4b) und einem Innenteil (4a), ausgebildet ist, oder
b) aus einer Litze (13a) mit metallisch leitendem Kontakt (13b) und einem Silberblech als Sensor (13c) besteht, wobei der Sensor (13c) zwischen der vliesartigen Unterlage (7') und dem Trägermaterial (1') angeordnet ist und durch diese vollständig überdeckt ist, und auf der hautabgewandten Seite des Trägermaterials (1') eine elektrisch isolierende Abdeckung (14) aufgeklebt ist, oder
c) aus einem Ag/AgCl-beschichteten Kunststoffteil mit einer angeformten Nase besteht, die das Trägermaterial (1, 1', 1") überragt und die mit einer Öffnung zur Befestigung einer Litze versehen ist, wobei gegebenenfalls das Kunststoffteil röntgentransluzent und das Trägermaterial (7, 7', 7") transparent ausgebildet ist, oder
d) als einteiliges Bauteil ausgebildet ist oder aus einem Kontaktelement besteht, an das jeweils durch plastische Verformung eine Litze aus isolierten, metallisch leitenden Seelen, wie Kupferdraht oder einer Kohlenstoffaser, angeschlagen ist.

7. Einmalelektrode nach einem der Ansprüche 1 bis 6, **dadurch gekennzeichnet, daß** die Grundfläche des Trägermaterials (1) größer ist als die Grundfläche der vliesartigen Unterlage (7) und die Fläche zwischen der Außenkontur und der vliesartigen Unterlage (7) und der Außenkontur des Trägermaterials (1) mit einem biokompatiblen Haftklebstoff (2) beschichtet ist, oder das Trägermaterial (1') und die vliesartige Unterlage (7') nahezu deckungsgleich ausgebildet sind und das Trägermaterial (1') hautseitig selbstklebend ausgerüstet ist, wobei die vliesartige Unterlage (7') auf der zur Haut zeigenden Seite des Trägermaterials (1') aufgeklebt ist.

8. Verfahren zur Herstellung einer Einmalelektrode nach Anspruch 1 durch mindestens folgende Verfahrensschritte in der angegebenen Reihenfolge:
a) Zuführung eines Trägermaterials (1, 1', 1"), dessen hautzugewandte Seite ganzflächig mit einem hautverträglichen Klebstoff (2, 2', 2") beschichtet ist, zu einer Montagevorrichtung, wobei die hautzugewandte Seite nach oben zeigt,
b) eine vorbereitete vliesartige Unterlage (7, 7', 7"), die in ihrer Grundfläche mindestens der Grundfläche des zu bildenden Leitgelkörpers (10, 10', 10") entspricht, wird auf der klebstoffbeschichteten Seite des Trägermaterials (1, 1', 1") befestigt,
c) auf die für eine einzelne Elektrode bestimmte, mit dem Trägermaterial verbundene vliesartige Unterlage(7, 7', 7") wird eine fließfähige Mischung zur Bildung des Leitgelkörpers (10, 10', 10") aufgetragen, wobei die Viskosität der fließfähigen Mischung derart hoch ist, dass deren Ausbreitung durch die äußere Kontur der Unterlage (7, 7', 7") begrenzt wird und die Fasern der Oberfläche der Unterlage (7, 7', 7") in die Mischung ragen,
d) die Mischung mit einem elektrisch leitenden Kontakt- oder Anschlußelement (4, 13) verbunden wird,
e) die Mischung zur Bildung des Gelkörpers (10, 10', 10") in an sich bekannter Weise zur Aushärtung oder Verfestigung gebracht wird, wobei zwischen den Fasern der Unterlage (7, 7', 7") und dem Leitgelkörper (10, 10', 10") ein Haftverbund gebildet wird, und
f) auf die zur Haut zeigende Seite der Elektrode eine abnehmbare, den Leitgelkörper (10, 10' 10") überdeckende Abdeckung (11, 11', 11") aufgeklebt wird.

9. Verfahren zur Herstellung einer Einmalelektrode nach Anspruch 2 durch mindestens folgende Verfahrensschritte in der angegebenen Reihenfolge:
a) Zuführung eines Trägermaterials (1, 1', 1"), dessen hautzugewandte Seite ganzflächig mit einem hautverträglichen Klebstoff (2, 2', 2") beschichtet ist, zu einer Montagevorrichtung, wobei die hautzugewandte Seite nach oben zeigt,
b) eine vorbereitete vliesartige Unterlage (7, 7', 7"), die in ihrer Grundfläche mindestens der Grundfläche des zu bildenden Leitgelkörpers (10, 10', 10") entspricht, wird auf der klebstoffbeschichteten Seite des Trägermaterials (1, 1', 1") befestigt,
c) auf die für eine einzelne Elektrode bestimmte, mit dem Trägermaterial verbundene vliesartige Unterlage (7, 7', 7") wird eine fließfähige Mischung zur Bildung des Leitgelkörpers (10, 10', 10") aufgetragen, wobei die Ausbreitung der Mischung durch eine parallel zur Außenkontur angeordnete oder mit der Außenkontur verlaufende, umlaufende Erhebung (8) der vliesartigen Unterlage (7, 7', 7") begrenzt wird und die Fasern der Oberfläche der Unterlage (7, 7', 7") in die Mischung ragen,
d) die Mischung mit einem elektrisch leitenden Kontakt- oder Anschlußelement (4,13) verbunden wird,
e) die Mischung zur Bildung des Gelkörpers (10, 10', 10") in an sich bekannter Weise zur Aushärtung oder Verfestigung gebracht wird, wobei zwischen den Fasern der Unterlage (7, 7', 7") und dem Leitgelkörper (10, 10', 10") ein Haftverbund gebildet wird, und
f) auf die zur Haut zeigende Seite der Elektrode eine abnehmbare, den Leitgelkörper (10, 10' 10") überdeckende Abdeckung (11, 11', 11") aufgeklebt wird.

10. Verfahren nach einem der Ansprüche 8 oder 9, **dadurch gekennzeichnet, daß** zwischen der vliesartigen Unterlage (7, 7', 7") und dem Trägermaterial (1,1', 1") eine steife Zwischenlage (5) angeordnet wird, die in ihrer Abmessung mindestens so groß ist wie die vliesartige Unterlage (7, 7', 7").

11. Verfahren nach einem der Ansprüche 8 bis 10, **dadurch gekennzeichnet, daß** die vliesartige Unterlage (7") mit über die Fläche verteilten Durchbrüchen (17) ausgerüstet und auf einem elektrisch leitfähigen Trägermaterial (7") aufgeklebt wird, und am Rand der Elektrode auf das Trägermaterial (7") ein umlaufender Schaumstoffstreifen (15) zur Verhinderung eines direkten elektrischen Kontaktes zur Haut über die Schnittkante des leitfähigen Trägermaterials (7") aufgeklebt wird, und die Elektrode mit einer seitlich abstehenden auf der hautzugewandten Seite elektrisch isolierten Lasche (12') als elektrisches Abgriffelement, an das eine Litze angeschlagen wird, ausgerüstet wird.

12. Verfahren nach einem der Ansprüche 8 bis 11, **dadurch gekennzeichnet, daß** die Elektrodenbauteile Trägermaterial (1, 1', 1") und vliesartige Unterlage (7, 7', 7") oder Trägermaterial (1, 1', 1"), vliesartige Unterlage (7, 7', 7") und die steife Unterlage (5), als separate Baueinheit vorgefertigt werden, die der weiteren Elektrodenmontage zugeführt wird.

13. Verfahren nach einem der Ansprüche 8 bis 12, **dadurch gekennzeichnet, daß** der mit der vliesartigen Unterlage (7, 7', 7") in inniger Verbindung stehende Leitgelkörper (10, 10', 10") aus einem in situ thermisch oder durch Strahlung initiierten Polymerisations- und Vernetzungsvorgang oder einer durch Wasserstoffbrückenbildung verursachten Verfestigung einer Reaktionsmischung oder durch Abkühlung einer durch Wärmezufuhr verflüssigbaren Mischung gebildet wird.

14. Verfahren nach einem der Ansprüche 8 bis 13, **dadurch gekennzeichnet, daß** die vliesartige Unterlage (7, 7', 7") vor dem Auftragen der Mischung zur Bildung des Leitgelkörpers (10, 10', 10") mit einer Flüssigkeit benetzt wird.

## Claims

1. Bio-medical disposable electrode, consisting of at least one conductive gel body (10, 10', 10") applied in a production line in free-flowing consistency and subsequently solidified, which, after application of said electrode, adheres to the patient's skin, a carrier material (1, 1') covering the conductive gel body (10, 10', 10") where the skin-facing side of said gel body is of planar shape; a removable cover (11, 11', 11") on the skin-facing side of the electrode, and an electrical connecting element (4, 13) connected with the conductive gel body (10, 10', 10"), **characterized in that** a planar pad with a fleece-like structure (7, 7', 7") is connected with the carrier material, with a reaction mixture being applied in free-flowing consistency to the skin-facing side of said carrier material for the formation of said gel body (10, 10', 10"), where the viscosity of said reaction mixture is such that its spread is limited by the outer contour of said fleece-like pad (7, 7', 7").

2. Bio-medical disposable electrode, consisting of at least one conductive gel body (10, 10', 10") applied in a production line in free-flowing consistency and subsequently solidified, which, after application of the electrode, adheres to the patient's skin; a carrier material (1,1') covering the conductive gel body (10, 10', 10") where the skin-facing side of said gel body is of planar shape; a removable cover (11, 11', 11") on the skin-facing side of the electrode; and an electrical connecting element (4, 13) connected with the conductive gel body (10, 10', 10"), **characterized in that** a planar pad with a fleece-like structure (7, 7', 7") is connected with the carrier material, with a reaction mixture being applied in free-flowing consistency to the skin-facing side of said carrier material for the formation of said gel body (10, 10', 10"), where the viscosity of said reaction mixture is such that its spread is limited by the outer contour of said fleece-like pad (7, 7', 7") and where said fleece-like pad (7, 7', 7") has a circumferential elevation arranged parallel to the outer contour or following the outer contour (8).

3. Disposable electrode according to any of claims 1 or 2 **characterized in that** the fleece-like pad (7, 7', 7") consists of organic artificial fibres or natural fibres or of mixtures of these fibres or of cellulose-containing material such as paper or cardboard, or a plastics layer with floccules on one side, and where the protruding free fibres of said fleece-like pad are firmly anchored in the gel body (10, 10', 10"), and where said fleece-like pad (7, 7', 7") has a mass per unit area of 4 to 250 g/m² and is consolidated either thermally or with the help of binding agents, and is at least partially permeable to liquids or UV radiation on the gel side.

4. Disposable electrode according to any of claims 1 to 3 **characterized in that** the carrier material (1, 1', 1") consists of a closed-cell polymer foam sheeting, a fabric made of woven or non-woven material or a compact plastics sheeting whose skin-facing side is coated with a bio-compatible adhesive material (2, 2', 2").

5. Disposable electrode according to any of claims 1 to 4 **characterized in that** the fleece-like pad (7, 7', 7") is firmly connected with an intermediate layer (5), by thermal force-fit and/or by means of a binding agent, and where the composite material is fastened on the skin-facing side of the carrier material (1) and said intermediate layer (5) consists of paper, cardboard or plastics and is provided with a circumferential elevation (5b) which has been adapted to exactly fit onto said elevation (8) of said fleece-like pad (7).

6. Disposable electrode according to any of claims 1 to 5 **characterized in that** the connecting element
a) is formed as a two-part push-button connection (4), consisting of an external part (4b) and an internal part (4a), or
b) consists of a strand (13a) with a metallically conductive contact (13b) and a silver plate as sensor (13c), where said sensor (13c) is arranged between the fleece-like pad (7') and the carrier material (1') and completely covered by it, and where on the skin-opposite side of said carrier material (1') an electrically insulating cover (14) has been pasted on, or
c) consists of an Ag/AgCl-coated plastics part with an integral nose which protrudes beyond the carrier material (1, 1', 1") and is provided with an opening for the fastening of the strand, where, if required, said plastics part is x-ray translucent and said carrier material (7, 7', 7") is transparent, or
d) is shaped as a single-part component or consists of a contact element to which a stand from insulated, metallically conductive cores such as copper wire or carbon fibre, is attached through plastic deformation.

7. Disposable electrode according to any of claims 1 to 6 **characterized in that** the area of the carrier material (1) is larger than the area of the fleece-like pad (7) and where the area between the outer contour and said fleece-like pad (7) and the outer contour of said carrier material (1) is coated with a bio-compatible pressure-sensitive adhesive (2) or said carrier material (1') and said fleece-like pad (7') are nearly congruent and said carrier material (1') is self-adhesive on the skin side, and where said fleece-like pad (7') is pasted onto the skin-facing side of said carrier material (1').

8. Method for the production of a disposable electrode according to claim 1 **characterized by** at least the following process steps in the sequence as given:
a) A carrier material (1, 1', 1"), whose skin-facing side is fully coated with a skin-compatible adhesive (2, 2', 2"), is fed to an assembly device, where said skin-facing side is pointing upwards;
b) A prepared fleece-like pad (7, 7', 7"), the area of which is at least as big as the area of the conductive gel body (10, 10', 10") to be formed, is fastened to the adhesive-coated side of the carrier material (1, 1', 1");
c) A free-flowing mixture for the formation of the conductive gel body (10, 10', 10") is applied to the fleece-like pad (7, 7', 7") designed for a single electrode and connected with the carrier material, with the viscosity of said free-flowing mixture being high enough to ensure that its spread is limited by the outer contour of the said pad (7, 7', 7") and the fibres on the surface of said pad (7, 7', 7") protrude into the mixture;
d) The mixture is connected with an electrically conductive contact or connecting element (4, 13);
e) The mixture for the formation of the gel body (10, 10', 10") is solidified or consolidated in a generally well-known way, with an adhesive bond being formed between the fibres of the pad (7, 7', 7") and said conductive gel body (10, 10', 10") ; and
f) A removable cover (11, 11', 11") fully covering the conductive gel body (10, 10' 10") is pasted onto the skin-facing side of the electrode.

9. Method for the production of a disposable electrode according to claim 2 **characterized by** at least the following process steps in the sequence as given:
a) A carrier material (1, 1', 1"), whose skin-facing side is fully coated with a skin-compatible adhesive (2, 2', 2"), is fed to an assembly device, where said skin-facing side is pointing upwards;
b) A prepared fleece-like pad (7, 7', 7"), the area of which is at least as big as the area of the conductive gel body (10, 10', 10") to be formed, is fastened to the adhesive-coated side of the carrier material (1, 1', 1");
c) A free-flowing mixture for the formation of the conductive gel body (10, 10', 10") is applied to the fleece-like pad (7, 7', 7") designed for a single electrode and connected with the carrier material, with the viscosity of the free-flowing mixture being high enough to ensure that its spread is limited by a circumferential elevation arranged parallel to the outer contour or together with the outer contour of said fleece-like pad (7, 7', 7") and the fibres on the surface of said pad (7, 7', 7") protrude into the mixture;
d) The mixture is connected with an electrically conductive contact or connecting element (4,13);
e) The mixture for the formation of the gel body (10, 10', 10") is solidified or consolidated in a generally well-known way, with an adhesive bond being formed between the fibres of the pad (7, 7', 7") and said conductive gel body (10, 10', 10") ; and
f) A removable cover fully covering the conductive gel body (10, 10' 10") is pasted onto the skin-facing side of the electrode (11, 11', 11").

10. Method according to any of claims 8 or 9 **characterized in that** a stiff intermediate layer (5) is arranged between the fleece-like pad (7, 7', 7") and the carrier material (1, 1', 1"), with the dimensions of said intermediate layer being at least as big as the fleece-like support (7, 7', 7").

11. Method according to any of claims 8 to 10 **characterized in that** the fleece-like support (7") is equipped with apertures (17) distributed across the area, and pasted onto an electrically conductive carrier material (7"), and where a circumferential foam material strip (15) is pasted onto said carrier material (7") on the edge of said electrode to prevent direct electrical contract with the skin across the cutting edge of said conductive carrier material (7"), and where said electrode is equipped with a laterally protruding lug (12') electrically insulated on the skin-facing side, as an electrical tap element to which a stand is attached.

12. Method according to any of claims 8 to 11 **characterized in that** the electrode components - carrier material (1, 1', 1") and fleece-like pad (7, 7', 7") - or carrier material (1, 1', 1"), fleece-like pad (7, 7', 7") and the stiff support (5) - are prefabricated as separate subassemblies and then fed to the subsequent electrode assembly process.

13. Method according to any of claims 8 to 12 **characterized in that** the conductive gel body (10, 10', 10") intimately connected with the fleece-like pad (7, 7', 7") is formed by an in-situ thermally initiated or radiation-initiated polymerization or cross-linking process or a solidification of a reaction mixture caused by hydrogen bridge formation or by cooling down a mixture liquefiable through heat supply.

14. Method according to any of claims 8 to 13 **characterized in that** the fleece-like pad (7, 7', 7") is wetted with a liquid before the mixture for the formation of the conductive gel body (10, 10', 10") is applied.

## Revendications

1. Électrode biomédicale à usage unique, composée d'au moins un élément de gel conducteur (10, 10', 10") qui passe au cours d'une ligne de fabrication d'une consistance coulante à une consistance finale solide et qui, après l'application de l'électrode, est en contact avec la peau du patient, d'un matériau support (1,1') recouvrant l'élément de gel conducteur (10, 10', 10") dont la face dirigée vers la peau est réalisée en face plane, d'un recouvrement amovible (11, 11', 11") situé sur la face de l'électrode tournée vers la peau et d'un connecteur électrique (4, 13) relié à l'élément de gel conducteur (10, 10', 10"), **caractérisée par** un support (7, 7', 7") plan non-tissé relié au matériau support (1, 1') sur la face tournée vers la peau duquel est appliqué un mélange réactionnel de consistance coulante pour former l'élément de gel (10, 10', 10"), la viscosité du mélange réactionnel étant tellement élevée que sa répartition est limitée par le contour extérieur du support non-tissé (7, 7', 7").

2. Électrode biomédicale à usage unique, composée d'au moins un élément de gel conducteur (10, 10', 10") qui passe au cours d'une ligne de fabrication d'une consistance coulante à une consistance finale solide et qui, après l'application de l'électrode, est en contact avec la peau du patient, d'un matériau support (1,1') recouvrant l'élément de gel conducteur (10, 10', 10") dont la face dirigée vers la peau est réalisée en face plane, d'un recouvrement amovible (11, 11', 11") situé sur la face de l'électrode tournée vers la peau et d'un connecteur électrique (4, 13) relié à l'élément de gel conducteur (10, 10', 10"), **caractérisée par** un support (7, 7' ,7") plan non-tissé relié au matériau support (1,1') sur la face tournée vers la peau duquel est appliqué un mélange réactionnel de consistance coulante pour former l'élément de gel (10, 10', 10"), mélange réactionnel dont la répartition est limitée par le contour extérieur du support non-tissé (7, 7' ,7"), le support non-tissé (7, 7' ,7") possédant un bord relevé (8) continu parallèle au contour extérieur ou parcourant tout le contour extérieur.

3. Électrode à usage unique selon l'une quelconque des revendications 1 ou 2, **caractérisée en ce que** le support non-tissé (7 ,7', 7") se compose de fibres organiques naturelles ou artificielles ou de mélanges de ces fibres ou d'une matière contenant de la cellulose, comme le papier ou le carton, ou d'une couche de plastique floqué sur une seule face, dont les fibres libres qui dépassent sont ancrées solidement à l'intérieur de l'élément de gel (10, 10', 10"), et le support non-tissé (7 ,7' ,7") présente un grammage compris entre 4 et 250 g/m² et fait l'objet d'une fixation thermique ou par liants en étant, du côté du gel, au moins partiellement perméable au liquide et aux rayons UV.

4. Électrode à usage unique selon l'une quelconque des revendications 1 à 3, **caractérisée en ce que** le matériau support (1, 1', 1") se compose d'une feuille de mousse polymère à cellules fermées, d'une surface textile composée d'un tissé ou d'un non-tissé ou d'une feuille de matière plastique compacte dont la face tournée vers la peau est recouverte d'une couche de colle (2, 2', 2") biocompatible.

5. Électrode à usage unique selon l'une quelconque des revendications 1 à 4, **caractérisée en ce que** le support non-tissé (7, 7' ,7") est fixé solidairement à un support intermédiaire (5) par adhésion thermique et/ou par liants, et le composé est fixé sur la face du matériau support (1) tournée vers la peau, et le support intermédiaire (5) se compose de papier, de carton ou de matière plastique et est pourvu d'un bord relevé continu (5b) dont la forme coïncide exactement avec le bord relevé (8) du support non-tissé (7).

6. Électrode à usage unique selon l'une quelconque des revendications 1 à 5, **caractérisée en ce que** le connecteur
a) est réalisé en connecteur à bouton-pression à deux éléments composé d'un élément extérieur (4b) et d'un élément intérieur (4a), ou
b) est composé d'un fil torsadé (13a) pourvu d'un contact de métal conducteur (13b) et d'une tôle d'argent servant de capteur (13c), le capteur (13c) étant disposé entre le support non-tissé (7') et le matériau support (1') en étant entièrement recouvert par ceux-ci, et un recouvrement (14) isolant électriquement est collé sur la face du matériau support (1') opposée à la peau, ou
c) se compose d'un élément de matière plastique recouvert de Ag/AgCl pourvu d'une came formée sur celui-ci qui dépasse du matériau support (1, 1', 1") et qui est pourvue d'un orifice pour fixer un fil torsadé, l'élément de matière plastique étant le cas échéant perméable aux rayons X et le matériau support (7, 7' ,7") transparent, ou
d) est réalisé en une seule pièce ou se compose d'un élément de contact sur lequel est monté à chaque fois, par déformation plastique, un fil torsadé composé de conducteurs métalliques isolés, comme les fils de cuivre ou les fibres de carbone.

7. Électrode à usage unique selon l'une quelconque des revendications 1 à 6, **caractérisée en ce que** la surface de base du matériau support (1) est supérieure à la surface de base du support non-tissé (7) et la surface située entre le contour extérieur et le support non-tissé (7) et le contour extérieur du matériau support (1) est recouverte d'une couche de colle biocompatible (2), ou le matériau support (1') et le support non-tissé (7') sont de forme presque égale et le matériau support (1') est autocollant sur la face tournée vers la peau, le support non-tissé (7') étant collé sur la face du matériau support (1') tournée vers la peau.

8. Procédé pour la fabrication d'une électrode à usage unique selon la revendication 1 au moins d'après les étapes de procédé suivantes réalisées dans l'ordre suivant:
a) Amenée d'un matériau support (1, 1', 1"), dont la face tournée vers la peau est recouverte sur toute sa surface d'une colle (2, 2', 2") haute tolérance pour l'épiderme, dans un dispositif d'assemblage, la face tournée vers la peau étant dirigée vers le haut,
b) un support non-tissé préfabriqué (7, 7', 7"), dont la surface de base correspond au moins à la surface de base de l'élément de gel conducteur (10, 10', 10") à créer, est fixé sur la face du matériau de support (1, 1', 1") recouverte d'une couche de colle,
c) un mélange coulant destiné à créer l'élément de gel conducteur (10, 10', 10") est appliqué sur le support non-tissé (7, 7', 7") relié au matériau support et destiné à une seule électrode, la viscosité du mélange coulant étant tellement élevée que sa répartition est limitée par le contour extérieur du support (7, 7', 7") et les fibres de la surface du support (7, 7', 7") pénètrent à l'intérieur du mélange,
d) le mélange est relié à un élément connecteur ou élément de contact (4, 13) conducteur de courant électrique,
e) le mélange destiné à créer l'élément de gel (10,10', 10") est porté à l'état durci ou solide d'une manière connue en soi, une adhérence étant créée entre les fibres du support (7 ,7', 7") et l'élément de gel conducteur (10, 10', 10"), et
f) un recouvrement (11, 11', 11 ") amovible recouvrant l'élément de gel conducteur (10, 10', 10") est collé sur la face de l'électrode tournée vers la peau.

9. Procédé pour la fabrication d'une électrode à usage unique selon la revendication 2 au moins d'après les étapes de procédé suivant réalisées dans l'ordre suivant:
a) Amenée d'un matériau support (1, 1', 1"), dont la face tournée vers la peau est recouverte sur toute sa surface d'une colle (2, 2', 2") haute tolérance pour l'épiderme, dans un dispositif d'assemblage, la face tournée vers la peau étant dirigée vers le haut,
b) un support non-tissé préfabriqué (7, 7', 7"), dont la surface de base correspond au moins à la surface de base de l'élément de gel conducteur (10, 10', 10") à créer, est fixé sur la face du matériau de support (1, 1', 1") recouverte d'une couche de colle,
c) un mélange coulant destiné à créer l'élément de gel conducteur (10, 10', 10") est appliqué sur le support non-tissé (7, 7', 7") relié au matériau support et destiné à une seule électrode, la répartition du mélange étant limitée par un bord relevé (8) continu parallèle au contour extérieur ou parcourant le contour extérieur du support non-tissé (7, 7', 7") et les fibres de la surface du support (7, 7', 7") pénètrent à l'intérieur du mélange,
d) le mélange est relié à un élément connecteur ou élément de contact (4, 13) conducteur de courant électrique,
e) le mélange destiné à créer l'élément de gel (10,10', 10") est porté à l'état durci ou solide d'une manière connue en soi, une adhérence étant créée entre les fibres du support (7, 7', 7") et l'élément de gel conducteur (10, 10', 10"), et
f) un recouvrement (11, 11', 11") amovible recouvrant l'élément de gel (10, 10', 10") est collé sur la face de l'électrode tournée vers la peau.

10. Procédé selon l'une quelconque des revendications 8 ou 9, **caractérisé en ce qu'**un support intermédiaire rigide (5) dont les dimensions sont au moins égales à celles du support non-tissé (7, 7', 7") est disposé entre le support non-tissé (7, 7', 7") et le matériau support (1, 1', 1 ").

11. Procédé selon l'une quelconque des revendications 8 à 10, **caractérisé en ce que** le support non-tissé (7") est équipé de percements (17) répartis sur toute la surface et est collé sur un matériau support (7") conducteur de courant électrique, et qu'une bande de mousse (15) continue destinée à empêcher un contact électrique direct avec la peau par l'intermédiaire de l'arête de coupe du matériau support conducteur (7") est collée au bord de l'électrode sur le matériau support (7"), et l'électrode est équipée d'une languette (12') isolée électriquement dépassant latéralement de la face tournée vers la peau et servant d'élément capteur électrique sur lequel est monté un fil torsadé.

12. Procédé selon l'une quelconque des revendications 8 à 11, **caractérisé en ce que** les composants de l'électrode, le matériau support (1, 1',1") et le support non-tissé (7, 7', 7") ou le matériau support (1,1', 1"), le support non-tissé (7, 7', 7") et le support rigide (5), sont préfabriqués sous forme de module séparé qui est amené à l'assemblage de l'électrode suivant.

13. Procédé selon l'une quelconque des revendications 8 à 12, **caractérisé en ce que** l'élément de gel conducteur (10, 10', 10") étroitement relié au support non-tissé (7, 7', 7") est créé par réticulation et polymérisation initiée thermiquement in situ ou par rayonnement ou par solidification d'un mélange réactionnel provoquée par liaison par ponts d'hydrogène ou par refroidissement d'un mélange liquéfiable par apport de chaleur.

14. Procédé selon l'une quelconque des revendications 8 à 13, **caractérisé en ce que** le support non-tissé (7, 7', 7") est humidifié à l'aide d'un liquide avant l'application du mélange destiné à créer l'élément de gel conducteur (10, 10', 10").
